(19) Europäisches Patentamt
European Patent Office
Office européen des brevets

(11) Veröffentlichungsnummer: **0 023 728**
**B1**

# (12) EUROPÄISCHE PATENTSCHRIFT

(45) Veröffentlichungstag der Patentschrift: **10.08.83**

(51) Int. Cl.³: **C 09 K 3/34,** C 07 C 121/75

(21) Anmeldenummer: **80200464.8**

(22) Anmeldetag: **19.05.80**

(54) **Anisotrope Verbindungen mit negativer oder positiver D.K. Anisotropie und geringer optischer Anisotropie und Flüssigkristallmischungen, die diese anisotropen Verbindungen enthalten.**

(30) Priorität: **18.07.79 CH 6671/79**

(43) Veröffentlichungstag der Anmeldung: **11.02.81 Patentblatt 81/6**

(45) Bekanntmachung des Hinweises auf die Patenterteilung: **10.08.83 Patentblatt 83/32**

(84) Benannte Vertragsstaaten: **CH DE FR GB LI**

(56) Entgegenhaltungen:
**DE-A-2 752 975**
**DE-A-2 854 310**
**DE-A-2 929 509**
**GB-A-2 017 742**
**US-A-4 029 595**

(73) Patentinhaber: **Merck Patent Gesellschaft mit beschränkter Haftung, Frankfurter Strasse 250, D-6100 Darmstadt (DE)**

(72) Erfinder: **Osman, Maged A., Dr.Ing.-Chem., Lerchenrain 1, CH-8046 Zürich (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 023 728 B1

ACTORUM AG

# Anisotrope Verbindungen mit negativer oder positiver DK-Anisotropie und geringer optischer Anisotropie und Flüssigkristallmischungen, die diese anisotropen Verbindungen enthalten

Die Erfindung betrifft neue anisotrope Verbindungen mit negativer oder positiver DK-Anisotropie und geringer, d.h. höchstens 0,2 betragender optischer Anisotropie (Δn), sowie Flüssigkristall(FK)mischungen, die diese anisotropen Verbindungen enthalten und als Dielektrikum für Flüssigkristallanzeigen verwendet werden.

Die bisher für Flüssigkristallmischungen bekannten anisotropen Verbindungen, deren DK-Anisotropie, kurz DKA oder Δε genannt, negativ ist, sind meist aus zwei oder drei aromatischen Ringen aufgebaute Verbindungen mit einer oder zwei Carboxylgruppen und gegebenenfalls einer Kovalenzbindung als Brücken zwischen den Ringen und mit einer oder mehreren Seitengruppen, d.h. nicht in der Moleküllängsachse liegenden kernständigen Substituenten, wie Nitril- oder Nitrogruppen, wie insbesondere in der DE-B Nr. 2240864, den DE-A Nrn. 2613293, 2835662 und 2836086 beschrieben. Diese bekannten anisotropen Verbindungen mit negativer DKA (Δε) haben aber hohe Viskositätswerte sowie hohe Werte der optischen Anisotropie (Δn); dabei ist $\Delta\varepsilon=\varepsilon_{//}-\varepsilon_{\perp}$ und $\Delta n=n_{//}-n_{\perp}$, d.h. die DKA einer Verbindung ist negativ, wenn die Dielektrizitätskonstante (ε) parallel zur Molekülachse kleiner ist als diejenige senkrecht zur Molekülachse. Die optische Anisotropie (Δn) einer Verbindung ist dann gross, wenn ihr Brechungsindex ($n_{\perp}$) senkrecht zur Molekülachse kleiner ist als ihr Brechungsindex ($n_{//}$) parallel zur Molekülachse; dieser optische Anisotropiewert, d.h. $\Delta n=n_{//}-n_{\perp}$ wird hier als gering bezeichnet, wenn er kleiner als oder höchstens 0,2 ($\Delta n\leqslant 0{,}2$) ist. Geeignete Methoden zur Messung von Δn sind aus der Literatur (z.B. I. Haller *et al.* in „Mol. Cryst. and Liqu. Cryst." *16*/1972/53-59) bekannt und werden meist auf eine bestimmte Lichtwellenlänge (hier 633 nm) bezogen.

Aus der Literatur, z.B. der DE-A Nr. 2636684, ist es bekannt, dass sich die Viskosität von flüssigkristallinen und linearen, d.h. keine Seitengruppen aufweisenden aromatischen Verbindungen, wie Biphenylnitrilen, vermindern lässt, indem einer der Phenylenkerne durch einen Cyclohexanring ersetzt wird. Für die herstellung von für FK-Mischungen geeigneten anisotropen Verbindungen mit grosser negativer DK-Anisotropie und kleinen Δn-Werten wurde diese Möglichkeit bisher noch nicht genutzt.

Aus DE-A Nr. 2752975 sind schliesslich dreikernige anisotrope Verbindungen mit zwei oder mehr Esterbrücken und ein oder zwei Cyclohexylringen in der Grundstruktur des Moleküls bekannt, das gegebenenfalls eine Seitengruppe tragen kann. Die Viskosität der Grundstruktur ist aber wegen der mindestens zweifach auftretenden Esterbrücke relativ hoch und die Seitengruppe gilt dabei nicht als kritisch. Anisotrope, d.h. enantiotropflüssigkristalline oder monotrope bzw. potentiell flüssigkristalline Verbindungen mit grosser negativer DK-Anisotropie und kleinen Δn-Werten werden aber, wie von der Anmelderin in der DE-C Nr. 2835863.6 beschrieben, für FK-Anzeigen benötigt, welche den sogenannten inversen *Guest-Host*-Effekt ausnützen; ferner werden anisotrope Verbindungen mit solchen Eigenschaften auch für bestimmte Typen von dynamischen Streuzellen benötigt. Die bisher bekannten anisotropen Verbindungen mit negativer DK-Anisotropie erfüllen die geforderten Bedingungen aber nicht oder nicht in befriedigendem Masse. Benötigt werden aber auch neue anisotrope Verbindungen mit positiver DKA und geringer optischer Anisotropie (Δn), z.B. für FK-Anzeigen mit Multiplexbetrieb. Ferner werden neue Verbindungen mit negativer oder positiver DKA, einer verringerten Viskosität und niedrigen γ-Werten ($\gamma=\Delta\varepsilon/\varepsilon_{\perp}$) benötigt.

Aufgabe der Erfindung ist es daher, eine neue Gruppe von anisotropen Verbindungen mit negativer oder positiver DKA, geringer optischer Anisotropie, niedrigen γ-Werten und verminderter Viskosität anzugeben.

Diese Aufgabe wird erfindungsgemäss gelöst durch anisotrope Verbindungen der Formel (1)

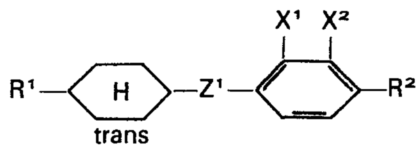
(1)

in der die Flügelgruppen $R^1$ und $R^2$ Wasserstoff, $C_1$ bis $C_{12}$-Alkylgruppen oder $C_1$ bis $C_{12}$-Alkoxygruppen sind, und eine der Gruppen $R^1$ und $R^2$ ausserdem einen cyclischen Rest der Formel (1a) oder (1b)

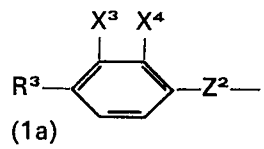
(1a)

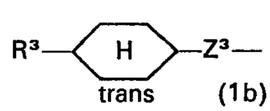
(1b)

darstellen kann; die Brücken $Z^1$, $Z^2$ und $Z^3$ bedeuten einfache Kovalenzbindungen oder Carboxyl- oder Oxycarbonylgruppen, wobei aber höchstens eine der Gruppen $Z^1$, $Z^2$, $Z^3$ Carboxyl bzw. Oxycarbonyl darstellt; $R^3$ ist Wasserstoff, $C_1$ bis $C_{12}$-Alkyl oder $C_1$ bis $C_{12}$-Alkoxy; von den Seitengruppen $X^1$ bis $X^4$ bedeutet mindestens eine Halogen, Nitril oder Nitro. Als Verbindungen mit negativer bzw. stark negativer DKA werden Verbindungen (1) bevorzugt, bei welchen zwei oder mehr der Seitengruppen $X^1$ bis $X^4$ Halogen, Nitro oder Nitril sind, wobei Nitril bevorzugt wird.

Beispiele für Halogen sind Chlor, Fluor, Brom und Jod. Es ist aber nicht erforderlich, dass alle $X^1$ bis $X^4$ als Seitengruppen wirken; dementsprechend können bis zu drei der Gruppen $X^1$ bis $X^4$, vorzugsweise bis zu zwei der Gruppen $X^1$ bis $X^4$, auch Wasserstoffatome darstellen.

Verbindungen (1) mit mehr als einer Estergruppe im Molekül werden nicht beansprucht.

Die erfindungsgemässen Verbindungen der Formel (1) werden im folgenden kurz auch als Verbindungen (1) bezeichnet.

Bevorzugte Gruppen der Verbindungen (1) entsprechen den folgenden Formeln (2) bis (6):

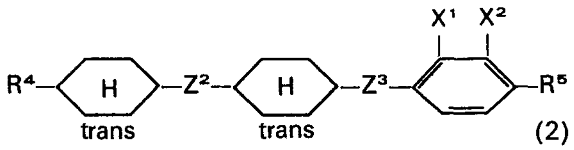
(2)

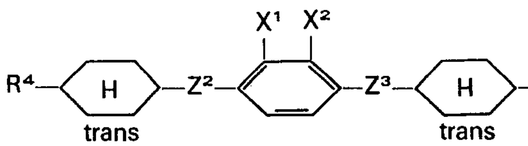
(3)

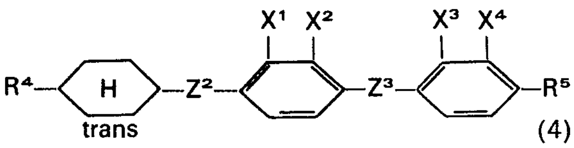
(4)

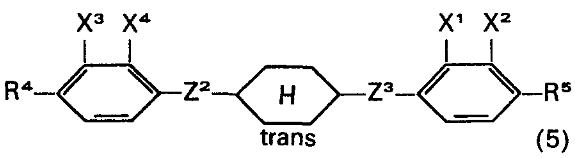
(5)

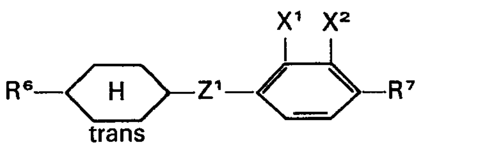
(6)

In den obigen Formeln (2) bis (6) bedeuten:
$R^4$ und $R^5$ Wasserstoff oder/und $C_1$ bis $C_{12}$-Alkyl oder/und $C_1$ bis $C_{12}$-Alkoxy, und
$R^6$ und $R^7$ $C_1$ bis $C_{12}$-Alkyl oder/und $C_1$ bis $C_{12}$-Alkoxy.

In allen Formeln (2) bis (6) sind für Verbindungen mit negativer DKA vorzugsweise mindestens zwei der Gruppen $X^1$ bis $X^4$ Halogen (vorzugsweise Chlor), Nitro oder Nitril, wobei Nitrilgruppen besonders bevorzugt werden. Gemäss einer ebenfalls bevorzugten Ausführungsform trägt einer der Benzolkerne der Verbindungen der Formeln (2) bis (6) zwei Halogenatome, Nitrogruppen oder (bevorzugt) Nitrilgruppen.

Vorzugsweise ist ferner in den Formeln (2) bis (5) eine der Brücken $Z^2$, $Z^3$ Carboxyl ($-COO-$) oder Oxycarbonyl ($-OOC-$) und die andere eine einfache Kovalenzbindung.

Die Verbindungen (1) können nach an sich bekannten Methoden erhalten werden, z.B. durch Kondensation von entsprechenden Phenolen bzw. Cyclohexanolen mit den entsprechenden Benzoesäure- bzw. Cyclohexancarbonsäureverbindungen, wobei die jeweilige Säurekomponente vorzugsweise in Form eines entsprechend reaktionsfähigen funktionellen Säurederivates, z.B. eines Säurehalogenides, insbesondere des Säurechlorides, eingesetzt und die Umsetzung in einer flüssigen organischen Base, wie Pyridin, durchgeführt wird. Spezielle Beispiele werden weiter unten gegeben.

Erfindungsgemässe FK-Mischungen enthalten als Komponente eine Verbindung (1), z.B. in Anteilen von 1 bis 40 Gew.-%, oder mehrere unterschiedliche Verbindungen (1) in einem gewünschtenfalls auch höheren Gesamtanteil. FK-Mischungen, die als Komponente mindestens eine Verbindung der obigen Formeln (2) bis (6) enthalten, werden bevorzugt.

Die Erfindung wird anhand der folgenden speziellen Beispiele weiter erläutert.

*Beispiel 1*

*Herstellung von 2-Cyano-4-n-butylphenyl-(4''-n-pentyl-trans-cyclohexyl-4')-trans-cyclohexanoat* (Formel 2; $R^4$=n$-C_5H_{11}-$, $Z^2$=—, $Z^3$=$-COO-$, $X^1$=$-CN$, $R^5$=n$-C_4H_9-$)

4-trans-(4'-trans-n-Pentylcyclohexyl)cyclohexancarbonsäure (7 g, 25 mmol) wurde mit Thionylchlorid (40 ml) 30 min auf Rückflusstemperatur erwärmt. Das entstandene Säurechlorid wurde vom überschüssigen Thionylchlorid befreit. Das so gewonnene Säurechlorid wurde nun zu einer Lösung von 2-Cyano-4-n-butylphenol (4,5 g, 25 mmol) in 100 ml Pyridin bei 0° C zugetropft. Nach Beendigung der Reaktion wurde das Gemisch in überschüssige verdünnte Salzsäure gegossen und mit Methylenchlorid extrahiert. Das aus dem Extrakt durch Eindampfen erhaltene Rohprodukt wurde aus Hexan umkristallisiert. Die so erhaltene Zielverbindung dieses Beispiels ist nematisch-enantiotrop ($T_m$=66,1° C, $T_c$=157,8° C) und hat eine negative DK-Anisotropie sowie ein $\Delta n \leqslant 0{,}2$.

*Beispiel 2*

*Herstellung von 2,3-Dichloro-4-n-pentylphenyl-(4''-n-pentyl-trans-cyclohexyl-4')-trans-cyclohexanoat* (Formel 2; $R^4$=n$-C_5H_{11}$, $Z^2$=—, $Z^3$=$-COO-$, $X^1$=Cl, $X^2$=Cl, $R^5$=n$-C_5H_{11}$)

4-trans-(4'-trans-n-Pentylcyclohexyl)cyclohexancarbonsäure (10 g, 36,5 mmol) wurde mit Thionylchlorid (50 ml) 1 h unter Rückfluss gekocht. Das überschüssige Thionylchlorid wurde destilliert. Das entstandene Säurechlorid wurde zu einer Lösung von 5,3 g (36,5 mmol) 2,3-Dichlor-4-n-pentylphenol (Kp. 85° C/0,007 Torr) in 100 ml Pyridin zugetropft. Die Reaktionsmischung wurde über Nacht gerührt und dann auf verdünnte Salzsäure gegossen. Das Produkt wurde mit Methylenchlorid extrahiert. Zur Reinigung wurde das Produkt aus Hexan umkristallisiert. Sie zeigt eine enantiotrope nematische Phase ($T_m$=40° C, $T_c$=180° C) und hat eine negative DK-Anisotropie sowie ein $\Delta n \leqslant 0{,}2$.

*Beispiel 3*

*Herstellung von 2,3-Dicyano-4-n-pentylphenyl-(4''-n-pentyl-trans-cyclohexyl-4')-trans-cyclohexanoat* (Formel 2; $R_4$=n$-C_5H_{11}$, $Z^2$=—, $Z^3$=$-COO-$, $X^1$=CN, $X^2$=CN, $R^5$=n$-C_5H_{11}$)

Nach der in Beispiel 2 beschriebenen Arbeitsweise wurde die Zielverbindung des vorliegenden Beispiels in analoger Weise aus äquimolaren (36,5 mmol) Mengen der in Beispiel 2 angegebenen Cyclohexancarbonsäureverbindung — wiederum unter Umwandlung derselben mit Thionylchlorid in das entsprechende Säurechlorid — und

2,3-Dicyano-4-n-pentylphenol (anstelle des entsprechenden Dichlorophenols von Beispiel 2) und Aufarbeitung wie oben hergestellt. Die so erhaltene Zielverbindung ist ebenfalls nematisch-enantiotrop und hat eine negative DK-Anisotropie sowie ein $\Delta n \leqslant 0{,}2$.

*Beispiel 4*

*Herstellung von 2-Cyano-4-n-butylphenyl-(4″-n-pentyl-trans-cyclohexyl-4′)-benzoat*
(Formel 4; $R^4 = n-C_5H_{11}$, $Z^2 = —$, $Z^3 = -COO-$, $X^1 = X^2 = X^4 = H$, $X^3 = CN$, $R^5 = n-C_4H_9$)

4-(4′-trans-n-Pentylcyclohexyl)benzoesäure (10 g, 36,5 mmol) wurde mit Thionylchlorid (50 ml) 1 h unter Rückfluss gekocht. Das entstandene Säurechlorid wurde von überschüssigem Thionylchlorid befreit. Das Säurechlorid wurde zu einer Lösung von 6,3 g (36,5 mmol) 2-Cyano-4-n-butylphenol in 100 ml Pyridin zugetropft. Nach Beendigung der Reaktion wurde das Gemisch in überschüssige verdünnte Salzsäure gegossen und mit Methylenchlorid extrahiert.

Das Produkt wurde aus der organischen Phase durch Eindampfen des Lösungsmittels erhalten. Zur Reinigung wurde das Rohprodukt aus Äthanol umkristallisiert. Die erhaltene Verbindung ist nematisch-flüssigkristallin ($T_m = 49{,}5°C$, $T_c = 115{,}5°C$) und hat eine negative DK-Anisotropie sowie ein $\Delta n \leqslant 0{,}2$.

*Beispiele 5 bis 22*

In ähnlicher Weise wie oben wurden die folgenden erfindungsgemässen anisotropen und eine negative DK-Anisotropie aufweisenden Verbindungen (1) hergestellt. Die Cyclohexylringe sind jeweils in trans-Konfiguration; $\Delta n \leqslant 0{,}2$:

5: 2,3-Dicyano-4-propylphenyl-(4″-propyl-p-phenylen-4′)-cyclohexanoat
6: 2,3-Dicyano-4-propylphenyl-(4″-pentyl-p-phenylen-4′)-cyclohexanoat
7: 2,3-Dicyano-4-propylphenyl-(2″,3″-dicyano-4″-propyl-p-phenylen-4′)-cyclohexanoat
8: 2,3-Dicyano-4-propylphenyl-(3″-cyano-4″-propyl-p-phenylen-4′)-cyclohexanoat
9: 2,3-Dichloro-4-pentylphenyl-(4″-propyl-p-phenylen-4′)-cyclohexanoat
10: 2,3-Dichloro-4-pentylphenyl-(3″-cyano-4″-propyl-p-phenylen-4′)-cyclohexanoat
11: 2,3-Dicyano-4-propylphenyl-(4″-pentyl-cyclohexyl-4′)-cyclohexanoat
12: 2,3-Dicyano-4-pentylphenyl-(4″-pentyl-cyclohexyl-4′)-cyclohexanoat
13: 2,3-Dichloro-4-propylphenyl-(4″-pentyl-cyclohexyl-4′)-cyclohexanoat
14: 2-Cyano-4-propylphenyl-(4″-pentylcyclohexyl-4′)-cyclohexanoat
15: 2-Cyano-4-pentylphenyl-(4″-pentylcyclohexyl-4′)-cyclohexanoat
16: 2-Chloro-4-pentylphenyl-(4″-pentyl-cyclohexyl-4′)-cyclohexanoat
17: 2-Nitro-4-pentylphenyl-(4″-pentylcyclohexyl-4′)-cyclohexanoat
18: 2-Cyano-4-propylphenyl-(4″-pentylcyclohexyl-4′)-benzoat
19: 2-Cyano-4-pentylphenyl-(4″-pentylcyclohexyl-4′)-benzoat
20: 2-Cyano-4-propylphenyl-(4″-pentylcyclohexyl-4′)-2′-cyanobenzoat
21: 2-Cyano-4-propylphenyl-(4″-pentylcyclohexyl-4′)-3′-cyanobenzoat
22: 2-Cyano-4-propylphenyl-(3″-cyano-4″-pentylphenylen-4′)-cyclohexanoat.

*Beispiele 23 bis 44*

Auch die folgenden erfindungsgemässen Verbindungen der obigen Formel (6) sind anisotrop und zeigen eine negative Anisotropie und ein $\Delta n \leqslant 0{,}2$:

23: 4-Propylcyclohexyl-4′-propyl-2′,3′-dicyanobenzoat
24: 4-Propylcyclohexyl-4′-pentyl-2′,3′-dicyanobenzoat
25: 2,3-Dicyano-4-propylphenyl-4′-heptylcyclohexanoat
26: 2,3-Dicyano-4-pentylphenyl-4′-propylcyclohexanoat
27: 2,3-Dicyano-4-pentylphenyl-4′-pentylcyclohexanoat
28: 2,3-Dicyano-4-propyloxyphenyl-4′-propylcyclohexanoat
29: 2,3-Dicyano-4-propyloxyphenyl-4′-pentylcyclohexanoat
30: 2,3-Dicyano-4-pentyloxyphenyl-4′-propylcyclohexanoat
31: 2,3-Dichloro-4-pentyloxyphenyl-4′-pentylcyclohexanoat
32: 2,3-Dichloro-4-propylphenyl-4′-propyloxycyclohexanoat
33: 2,3-Dibromo-4-pentylphenyl-4′-propoxycyclohexanoat
34: 2,3-Dinitro-4-pentylphenyl-4′-pentyloxycyclohexanoat
35: 2,3-Dicyano-4-propylphenyl-4′-propylcyclohexan
36: 2,3-Dicyano-4-propylphenyl-4′-pentylcyclohexan
37: 2,3-Dicyano-4-propylphenyl-4′-heptylcyclohexan
38: 2,3-Dicyano-4-pentyloxyphenyl-4′-propoxycyclohexan
39: 2,3-Dicyano-4-pentyloxyphenyl-4′-pentyloxycyclohexan
40: 2,3-Dicyano-4-propylphenyl-4′-butoxycyclohexan
41: 2,3-Dichloro-4-pentylphenyl-4′-propylcyclohexan
42: 2,3-Dinitro-4-heptylphenyl-4′-propylcyclohexan
43: 2,3-Dichloro-4-pentylphenyl-4′-butoxycyclohexan
44: 2,3-Dinitro-4-pentylphenyl-4′-butoxycyclohexan

Alle Verbindungen der Beispiele 1 bis 44 haben ausser einer negativen DK-Anisotropie auch vorteilhaft niedrige $\Delta n$-Werte $\leqslant 0{,}2$ und die Cyclohexanringe liegen jeweils in trans-Konfiguration vor. Die erfindungsgemässen Verbindungen (1) haben den überraschenden zusätzlichen Vorteil, dass ihre

γ-Werte ($\gamma = \Delta\varepsilon/\varepsilon_{\perp}$) klein sind und häufig höchstens etwa 2 betragen, d.h. $\gamma \leqslant 2$.

## Patentansprüche

1. Anisotrope Verbindungen mit negativer oder positiver DK-Anisotropie und einer optischen Anisotropie $\Delta n \leqslant 0{,}2$, gekennzeichnet durch die Formel (1)

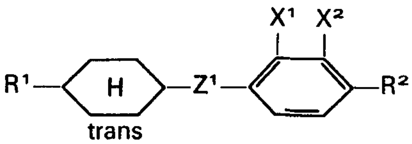
(1)

in der $R^1$ und $R^2$ Wasserstoffatome, Alkylgruppen mit 1 bis 12 C-Atomen oder Alkoxygruppen mit 1 bis 12 C-Atomen sind, und eine der Gruppen $R^1$ und $R^2$ ausserdem einen cyclischen Rest der Formel (1a) oder (1b)

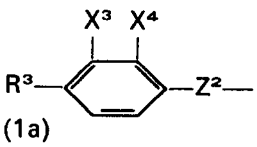
(1a)

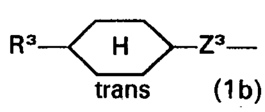
(1b)

darstellen kann, die Brücken $Z^1$, $Z^2$ und $Z^3$ gleich oder verschieden sind und einfache Kovalenzbindungen, Carboxyl- oder Oxycarbonylgruppen bedeuten, $R^3$ das Wasserstoffatom, eine Alkylgruppe mit 1 bis 12 C-Atomen oder eine Alkoxygruppe mit 1 bis 12 C-Atomen ist, und $X^1$ bis $X^4$ Wasserstoffatome, Halogenatome, Nitrilgruppen oder Nitrogruppen sind, wobei stets mindestens eine der Gruppen $X^1$ bis $X^4$ nicht das Wasserstoffatom ist und wobei höchstens eine der Brücken $Z^1$, $Z^2$, $Z^3$ eine Carboxyl- oder Oxycarbonylgruppe ist.

2. Verbindung nach Patentanspruch 1, gekennzeichnet durch die Formel (2)

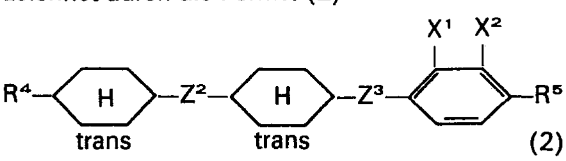
(2)

in der $R^4$ und $R^5$ Wasserstoffatome oder Alkyl- oder Alkoxygruppen mit jeweils 1 bis 12 C-Atomen bedeuten.

3. Verbindung nach Patentanspruch 1, gekennzeichnet durch die Formel (3)

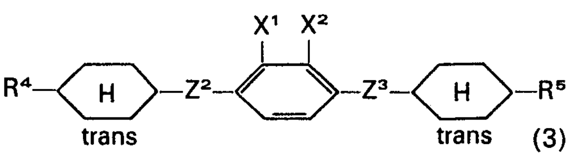
(3)

in der $R^4$ und $R^5$ Wasserstoffatome oder Alkyl- oder Alkoxygruppen mit jeweils 1 bis 12 C-Atomen bedeuten und $X^1$ und $X^2$ nicht Wasserstoffatome sind, wenn $Z^2$ die Carboxylgruppe ist.

4. Verbindung nach Patentanspruch 1, gekennzeichnet durch die Formel (4)

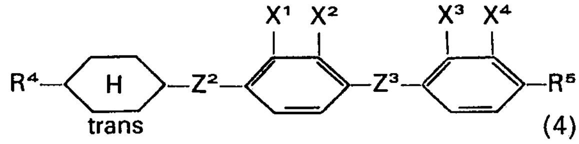
(4)

in der $R^4$ und $R^5$ Wasserstoffatome oder Alkyl- oder Alkoxygruppen mit jeweils 1 bis 12 C-Atomen bedeuten und mindestens zwei der Gruppen $X^1$ bis $X^4$ nicht Wasserstoffatome sind, wenn $Z^2$ die Carboxylgruppe bedeutet.

5. Verbindung nach Patentanspruch 1, gekennzeichnet durch die Formel (5)

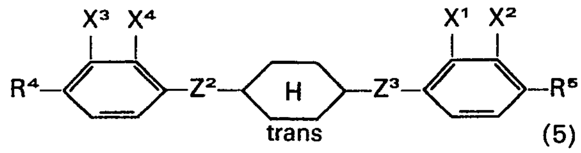
(5)

in der $R^4$ und $R^5$ Wasserstoffatome oder Alkyl- oder Alkoxygruppen mit jeweils 1 bis 12 C-Atomen bedeuten.

6. Verbindung nach Patentanspruch 1, gekennzeichnet durch die Formel (6)

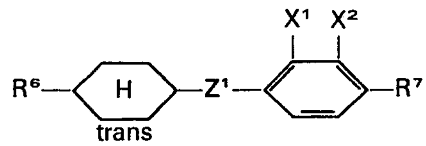
(6)

in der $R^6$ und $R^7$ Alkyl- oder/und Alkoxygruppen mit jeweils 1 bis 12 C-Atomen sind.

7. Verbindung nach einem der Patentansprüche 1 bis 6, dadurch gekennzeichnet, dass beide $X^1$ und $X^2$ Halogenatome, Nitrogruppen oder vorzugsweise Nitrilgruppen sind.

8. Verbindung nach einem der Patentansprüche 1 bis 5, dadurch gekennzeichnet, dass mindestens zwei der Gruppen $X^1$ bis $X^4$ Halogenatome, Nitrogruppen oder vorzugsweise Nitrilgruppen sind.

9. Flüssigkristallmischung, die als Komponente mindestens eine anisotrope Verbindung mit negativer oder positiver DK-Anisotropie und einer optischen Anisotropie $\Delta n \leqslant 0{,}2$ enthält, dadurch gekennzeichnet, dass die anisotrope Verbindung eine solche der Formel (1)

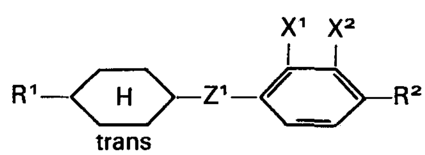
(1)

ist, in der $R^1$ und $R^2$ Wasserstoffatome, Alkylgruppen mit 1 bis 12 C-Atomen oder Alkoxygruppen mit 1 bis 12 C-Atomen sind, und eine der Gruppen $R^1$ und $R^2$ ausserdem einen cyclischen Rest der Formel (1a) oder (1b)

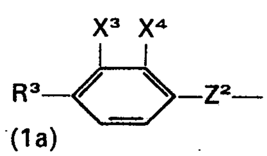
(1a)

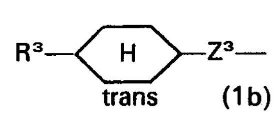
(1b)

darstellen kann, die Brücken $Z^1$, $Z^2$ und $Z^3$ gleich oder verschieden sind und einfache Kovalenzbindungen, Carboxyl- oder Oxycarbonylgruppen bedeuten, $R^3$ das Wasserstoffatom, eine Alkylgruppe mit 1 bis 12 C-Atomen oder eine Alkoxygruppe mit 1 bis 12 C-Atomen ist und $X^1$ bis $X^4$ Wasserstoffatome, Halogenatome, Nitrilgruppen oder Nitrogruppen sind, wobei stets mindestens eine der Gruppen $X^1$ bis $X^4$ nicht das Wassserstoffatom ist und wobei höchstens eine der Brücken $Z^1$, $Z^2$, $Z^3$ eine Carboxyl- oder Oxycarbonylgruppe ist.

10. FK-Mischung nach Patentanspruch 9, dadurch gekennzeichnet, dass die Verbindung (1) eine solche der Formel (2)

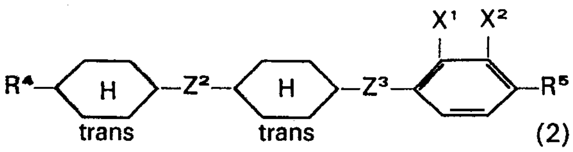
(2)

ist, in der $R^4$ und $R^5$ Wasserstoffatome oder Alkyl- oder Alkoxygruppen mit jeweils 1 bis 12 C-Atomen bedeuten.

11. FK-Mischung nach Patentanspruch 9, dadurch gekennzeichnet, dass die Verbindung (1) eine solche der Formel (3)

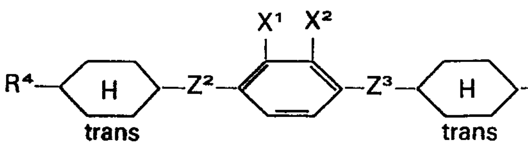
(3)

ist, in der $R^4$ und $R^5$ Wasserstoffatome oder Alkyl- oder Alkoxygruppen mit jeweils 1 bis 12 C-Atomen bedeuten und $X^1$ und $X^2$ nicht Wasserstoffatome sind, wenn $Z^2$ die Carboxylgruppe ist.

12. FK-Mischung nach Patentanspruch 9, dadurch gekennzeichnet, dass die Verbindung (1) eine solche der Formel (4)

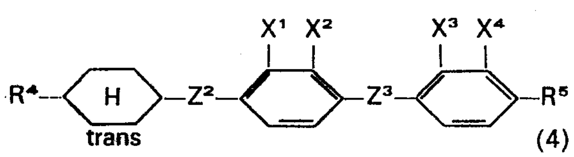
(4)

ist, in der $R^4$ und $R^5$ Wasserstoffatome oder Alkyl- oder Alkoxygruppen mit jeweils 1 bis 12 C-Atomen bedeuten und mindestens zwei der Gruppen $X^1$ bis $X^4$ nicht Wasserstoffatome sind, wenn $Z^2$ die Carboxylgruppe bedeutet.

13. FK-Mischung nach Patentanspruch 9, dadurch gekennzeichnet, dass die Verbindung (1) eine solche der Formel (5)

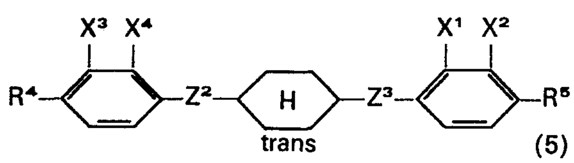
(5)

ist, in der $R^4$ und $R^5$ Wasserstoffatome oder Alkyl- oder Alkoxygruppen mit jeweils 1 bis 12 C-Atomen bedeuten.

14. FK-Mischung nach Patentanspruch 9, dadurch gekennzeichnet, dass die Verbindung (1) eine solche der Formel (6)

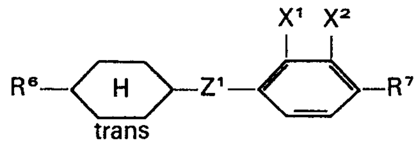
(6)

ist, in der $R^6$ und $R^7$ Alkyl- oder/und Alkoxygruppen mit jeweils 1 bis 12 C-Atomen sind.

15. FK-Mischung nach einem der Patentansprüche 9 bis 14, dadurch gekennzeichnet, dass beide $X^1$ und $X^2$ Halogenatome, Nitrogruppen oder vorzugsweise Nitrilgruppen sind.

16. FK-Mischung nach einem der Patentansprüche 11 bis 13, dadurch gekennzeichnet, dass mindestens zwei der Gruppen $X^1$ bis $X^4$ Halogenatome, Nitrogruppen oder vorzugsweise Nitrilgruppen sind.

## Revendications

1. Composés anisotropes à anisotropie diélectrique négative ou positive et anisotropie optique $\Delta n$ inférieure ou égale à 0,2, caractérisés par la formule 1:

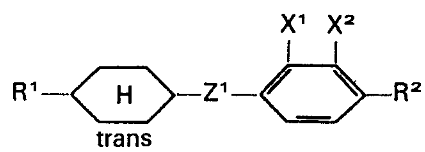
(1)

dans laquelle $R^1$ et $R^2$ représentent des atomes d'hydrogène, des groupes alkyle en C1-C12 ou des groupes alcoxy en C1-C12, l'un des symboles $R^1$ et $R^2$ pouvant en outre représenter un reste cyclique de formule 1a ou 1b:

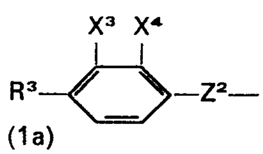
(1a)

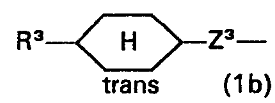
(1b)

les ponts $Z^1$, $Z^2$ et $Z^3$, identiques ou différents, consistant en liaisons de covalence simples, groupes carboxyle ou oxycarbonyle, $R^3$ représente un atome d'hydrogène, un groupe alkyle en C1-C12 ou alcoxy en C1-C12, et $X^1$ à $X^4$ représentent des atomes d'hydrogène, d'halogènes, des groupes nitriles ou nitro, l'un au moins des symboles $X^1$ à $X^4$ ayant en permanence une signification autre qu'un atome d'hydrogène, et au maximum un des ponts $Z^1$, $Z^2$, $Z^3$ consistant en un groupe carboxyle ou oxycarbonyle.

2. Composé selon la revendication 1, caractérisé par la formule 2:

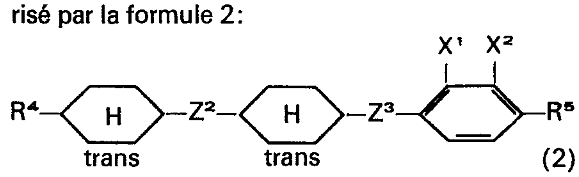
(2)

dans laquelle $R^4$ et $R^5$ représentent des atomes d'hydrogène ou des groupes alkyle ou alcoxy contenant chacun 1 à 12 atomes de carbone.

3. Composé selon la revendication 1, caractérisé par la formule 3:

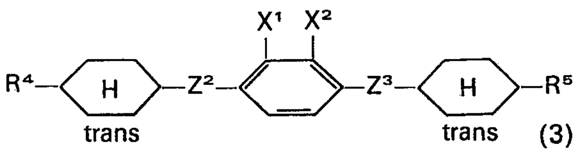

trans trans (3)

dans laquelle $R^4$ et $R^5$ représentent des atomes d'hydrogène ou des groupes alkyle ou alcoxy contenant chacun 1 à 12 atomes de carbone, et $X^1$ et $X^2$ ne représentent pas des atomes d'hydrogène lorsque $Z^2$ représente le groupe carboxyle.

4. Composé selon la revendication 5, caractérisé par la formule 4:

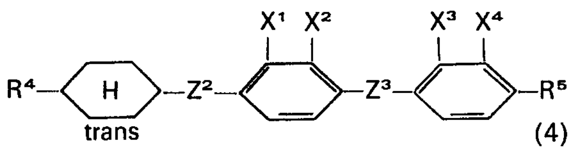

trans (4)

dans laquelle $R^4$ et $R^5$ représentent des atomes d'hydrogène ou des groupes alkyle ou alcoxy contenant chacun 1 à 12 atomes de carbone, deux au moins des symboles $X^1$ à $X^4$ ayant des significations autres que des atomes d'hydrogène lorsque $Z^2$ représente le groupe carboxyle.

5. Le composé selon la revendication 1, caractérisé par la formule 5:

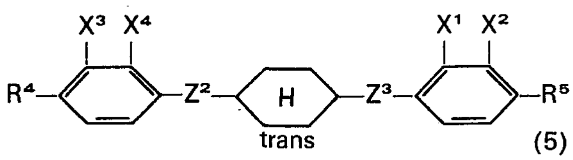

trans (5)

dans laquelle $R^4$ et $R^5$ représentent des atomes d'hydrogène ou des groupes alkyle ou alcoxy contenant chacun 1 à 12 atomes de carbone.

6. Composé selon la revendication 1, caractérisé par la formule 6:

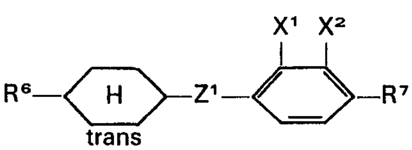

(6)

trans

dans laquelle $R^6$ et $R^7$ représentent des groupes alkyle et/ou alcoxy contenant chacun 1 à 12 atomes de carbone.

7. Composé selon l'une des revendications 1 à 6, caractérisé en ce que les deux symboles $X^1$ et $X^2$ représentent des atomes d'halogènes, des groupes nitro ou de préférence des groupes nitriles.

8. Composé selon l'une des revendications 1 à 5, caractérisé en ce que deux au moins des symboles $X^1$ à $X^4$ représentent des atomes d'halogènes, des groupes nitro ou de préférence des groupes nitriles.

9. Mélange de cristaux liquides contenant en tant que composant au moins un composé anisotrope à anisotropie diélectrique négative ou positive et anisotropie optique $\Delta n$ inférieure ou égale à 0,2, caractérisé en ce que le composé anisotrope est un composé de formule 1:

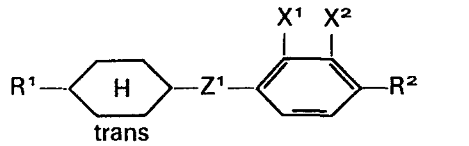

(1)

trans

dans laquelle $R^1$ et $R^2$ représentent des atomes d'hydrogène, des groupes alkyle en C1-C12 ou alcoxy en C1-C12, l'un des symboles $R^1$ et $R^2$ pouvant en outre représenter un reste cyclique de formule 1a ou 1b:

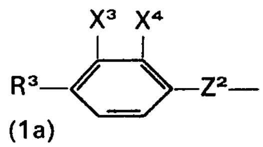

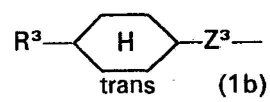

(1a) trans (1b)

les ponts $Z^1$, $Z^2$ et $Z^3$, identiques ou différents, consistant en liaisons de covalence simples, groupes carboxyle ou oxycarbonyle, $R^3$ représente l'atome d'hydrogène, un groupe alkyle en C1-C12 ou alcoxy en C1-C12, et $X^1$ à $X^4$ représentent des atomes d'hydrogène, d'halogènes, des groupes nitriles ou nitro, l'un au moins des symboles $X^1$ à $X^4$ ayant en permanence une signification autre qu'un atome d'hydrogène, et au maximum un des ponts $Z^1$, $Z^2$, $Z^3$, consistant en un groupe carboxyle ou oxycarbonyle.

10. Mélange de cristaux liquides selon la revendication 9, caractérisé en ce que le composé 1 est un composé de formule 2:

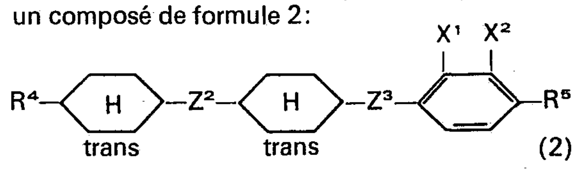

trans trans (2)

dans laquelle $R^4$ et $R^5$ représentent des atomes d'hydrogène, des groupes alkyle ou alcoxy contenant chacun 1 à 12 atomes de carbone.

11. Mélange de cristaux liquides selon la revendication 9, caractérisé en ce que le composé 1 est un composé de formule 3:

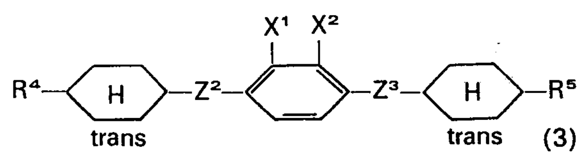

trans trans (3)

dans laquelle $R^4$ et $R^5$ représentent des atomes d'hydrogène ou des groupes alkyle ou alcoxy contenant chacun 1 à 12 atomes de carbone, et $X^1$ et $X^2$ ont des significations autres que les atomes d'hydrogène lorsque $Z^2$ représente le groupe carboxyle.

12. Mélange de cristaux liquides selon la revendication 9, caractérisé en ce que le composé 1 est un composé de formule 4:

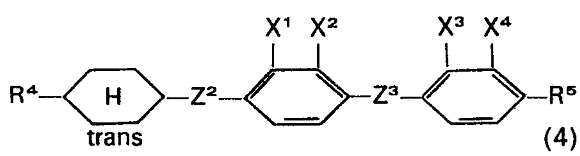

trans (4)

dans laquelle $R^4$ et $R^5$ représentent des atomes d'hydrogène ou des groupes alkyle ou alcoxy contenant chacun 1 à 12 atomes de carbone, deux au moins des symboles $X^1$ à $X^4$ ayant des significations autres que des atomes d'hydrogène lorsque $Z^2$ représente le groupe carboxyle.

13. Mélange de cristaux liquides selon la revendication 9, caractérisé en ce que le composé 1 est un composé de formule 5:

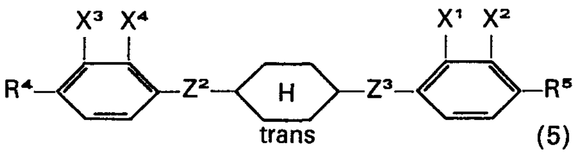

(5)

dans laquelle $R^4$ et $R^5$ représentent des atomes d'hydrogène ou des groupes alkyle ou alcoxy contenant chacun 1 à 12 atomes de carbone.

14. Mélange de cristaux liquides selon la revendication 9, caractérisé en ce que le composé 1 est un composé de formule 6:

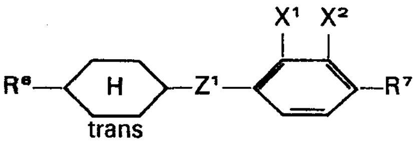

(6)

dans laquelle $R^6$ et $R^7$ représentent des groupes alkyle et/ou alcoxy contenant chacun 1 à 12 atomes de carbone.

15. Mélange de cristaux liquides selon l'une des revendications 9 à 14, caractérisé en ce que les deux symboles $X^1$ et $X^2$ représentent des atomes d'halogènes, des groupes nitro ou de préférence des groupes nitriles.

16. Mélange de cristaux liquides selon l'une des revendications 9 à 13, caractérisé en ce que deux au moins des symboles $X^1$ à $X^4$ représentent des atomes d'halogènes, des groupes nitro ou de préférence des groupes nitriles.

## Claims

1. An anisotropic compound with negative or positive DC-anisotropy and an optical anisotropy $\Delta n \leq 0.2$, having the formula (1)

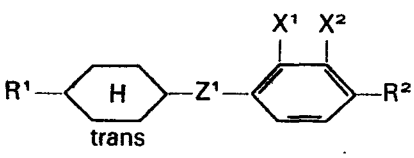

(1)

in which $R^1$ and $R^2$ are independently hydrogen, alkyl having 1 to 12 carbon atoms, alkoxy having 1 to 12 carbon atoms, or one of $R^1$ and $R^2$ can represent a cyclical fragment of the formula (1a) or (1b)

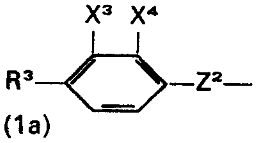

(1a)

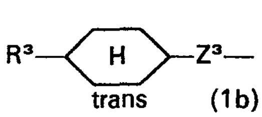

(1b)

the bridges $Z^1$, $Z^2$ and $Z^3$ are identical or different and are covalent bonds, carboxyl or oxycarbonyl groups, $R^3$ is hydrogen, alkyl of 1 to 12 carbon atoms or alkoxy of 1 to 12 carbon atoms, and $X^1$ to $X^4$ are independently hydrogen, halogen, nitrile or nitro, wherein at least one of $X^1$ to $X^4$ is not hydrogen and at most one of the bridges $Z^1$, $Z^2$ and $Z^3$ is a carboxyl or oxycarbonyl group.

2. The compound of claim 1, having the formula (2)

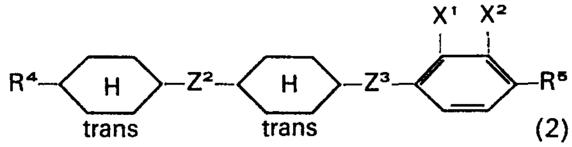

(2)

wherein $R^4$ and $R^5$ are hydrogen, alkyl or alkoxy groups having 1 to 12 carbon atoms, respectively.

3. The compound of claim 1, having the formula (3)

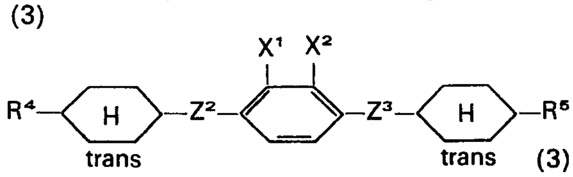

(3)

wherein $R^4$ and $R^5$ are hydrogen, alkyl or alkoxy groups having 1 to 12 carbon atoms, respectively, and $X^1$ and $X^2$ are not hydrogen when $Z^2$ is a carboxyl group.

4. The compound of claim 1, having the formula (4)

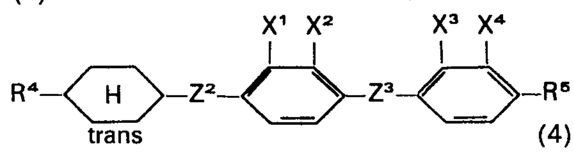

(4)

wherein $R^4$ and $R^5$ are hydrogen, alkyl or alkoxy groups having 1 to 12 carbon atoms, respectively, and at least two of the groups $X^1$ to $X^4$ are not hydrogen when $Z^2$ is carboxyl.

5. The compound of claim 1, having the formula (5)

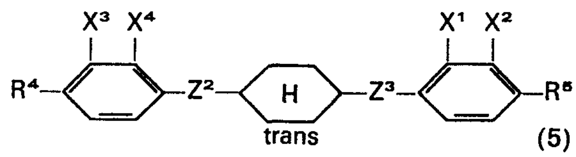

(5)

wherein $R^4$ and $R^5$ are hydrogen, alkyl or alkoxy groups having 1 to 12 carbon atoms, respectively.

6. The compound of claim 1, having the formula (6)

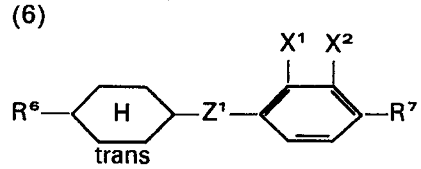

(6)

wherein $R^6$ and $R^7$ are alkyl or alkoxy groups having 1 to 12 carbon atoms, respectively.

7. The compound of claim 1, 2, 3, 4, 5 or 6, wherein both $X^1$ and $X^2$ are halogen, nitro or preferably nitrile.

8. The compound of claim 1, 2, 3, 4 or 5, wherein at least two of the groups $X^1$ to $X^4$ are halogen, nitro or preferably nitrile.

9. A liquid crystal composition which comprises as components at least one anisotropic compound with negative or positive DC-anisotropy and an optical anisotropy $\Delta n \leqslant 0.2$, wherein the anisotropic compound is a compound having the formula (1)

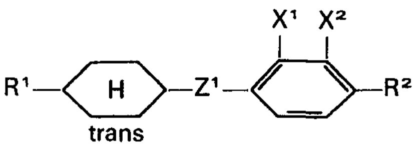

(1)

wherein $R^1$ and $R^2$ are independently hydrogen, alkyl having 1 to 12 cabon atoms, alkoxy having 1 to 12 carbon atoms, or one of $R^1$ and $R^2$ can represent a cyclical fragment of the formula (1a) or (1b)

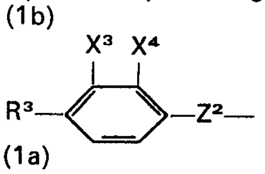

(1a)

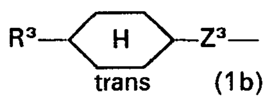

(1b)

the bridges $Z^1$, $Z^2$ and $Z^3$ are identical or different and are covalent bonds, carboxyl or oxycarbonyl groups, $R^3$ is hydrogen, alkyl of 1 to 12 carbon atoms or alkoxy of 1 to 12 carbon atoms, and $X^1$ to $X^4$ are independently hydrogen, halogen, nitrile or nitro, wherein at least one of $X^1$ to $X^4$ is not hydrogen and at most one of the bridges $Z^1$, $Z^2$ and $Z^3$ is a carboxyl or oxycarbonyl group.

10. The liquid crystal composition of claim 9, wherein the anisotropic compound has the formula (2)

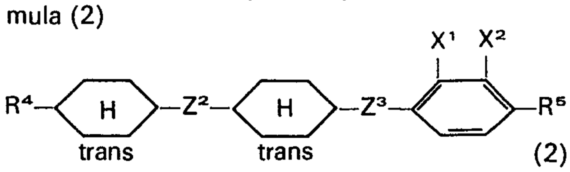

(2)

wherein $R^4$ and $R^5$ are hydrogen, alkyl or alkoxy having 1 to 12 carbon atoms, respectively.

11. The liquid crystal composition of claim 9, wherein the anisotropic compound has the formula (3)

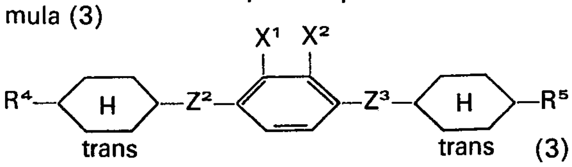

(3)

wherein $R^4$ and $R^5$ are hydrogen, alkyl or alkoxy having 1 to 12 carbon atoms, respectively, and $X^1$ and $X^2$ are not hydrogen when $Z^2$ is a carboxyl group.

12. The liquid crystal composition of claim 9, wherein the anisotropic compound has the formula (4)

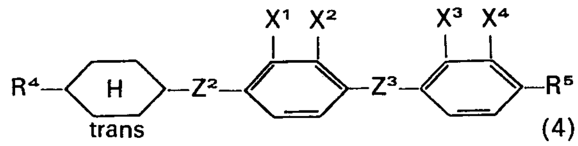

(4)

wherein $R^4$ and $R^5$ are hydrogen, alkyl or alkoxy having 1 to 12 carbon atoms, respectively, and at least two of $X^1$ to $X^4$ are not hydrogen when $R^2$ is carboxyl.

13. The liquid crystal composition of claim 9, wherein the anisotropic compound has the formula (5)

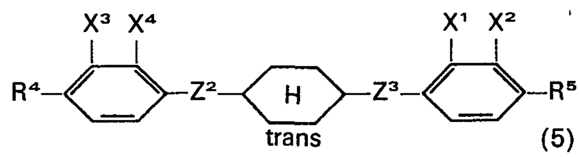

(5)

wherein $R^4$ and $R^5$ are hyrogen, alkyl or alkoxy having 1 to 12 carbon atoms, respectively.

14. The liquid crystal composition of claim 9, wherein the anisotropic compound has the formula (6)

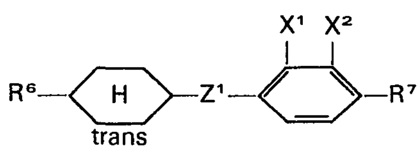

(6)

wherein $R^6$ and $R^7$ are alkyl or alkoxy having 1 to 12 carbon atoms, respectively.

15. The liquid crystal composition of claim 9, 10, 11, 12, 13 or 14, wherein both $X^1$ and $X^2$ are halogen, nitro or preferably nitrile.

16. The liquid crystal composition of claim 9, 10, 11, 12 or 13, wherein at least two of $X^1$ to $X^4$ are halogen, nitro or preferably nitrile.